# EUROPEAN PATENT APPLICATION

(11) **EP 3 323 415 A1**
(43) Date of publication of application: **23.05.2018**
(21) Application number: 16199152.6
(22) Date of filing: 16.11.2016
(51) Int. Cl.: A61K 31/53, A61K 31/7084, A61P 25/28

(54) **TREATMENT OF NEURODEGENERATIVE DISEASES**

(71) Applicant: Institut du Cerveau et de la Moelle Épinière-ICM, 75013 Paris (FR); UNIVERSITÈ PIERRE ET MARIE CURIE - PARIS 6 (UPMC), 75005 Paris (FR); CENTRE NATIONAL DE LA RECHERCHE SCIENTIFIQUE, 75016 Paris (FR); INSERM (Institut National de la Santé et de la Recherche Médicale), 75654 Paris Cedex 13 (FR); APHP (Assistance Publique - Hôpitaux de Paris), 75001 Paris (FR)
(72) Inventor: GUERREIRO DA SILVA, Serge, 75013 Paris (FR); MICHEL, Patrick Pierre, 75013 Paris (FR)
(74) Representative: Icosa

(57) **Abstract**

The present invention relates to an agonist of the receptor of 1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid for the treatment of neurodegenerative disorders.

## Description

### FIELD OF INVENTION

The present invention relates to the field of neurodegenerative diseases and in particular methods to treat neurodegenerative diseases. Especially, the present invention relates to the use of an agonist of the receptor of 1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid (hereafter referred to as "compound 1") for the treatment of such disorders.

### BACKGROUND OF INVENTION

Neurodegenerative disease is an umbrella term for a range of conditions which primarily affect the neurons in the human brain and spinal cord.

Neurons are the building blocks of the nervous system which includes the brain and spinal cord. Neurons normally don't reproduce or replace themselves, so when they become damaged or die they cannot be replaced by the body. Examples of neurodegenerative diseases include Parkinson's, Alzheimer's diseases, or amyotrophic lateral scleroses which result in progressive degeneration and / or death of nerve cells. Symptoms of such diseases relate to impairments of movement (called ataxias), or mental functioning (called dementias).

The process of neurodegeneration is not well-understood so the diseases that stem from it have, as yet, no cures. Medications, brain surgery, and multidisciplinary management are the therapeutic strategies used to provide relief from the symptoms in some patients. In the case of Parkinson's disease, levodopa has been the most widely used drug to reduce motor symptoms, however, its effect is temporary and invalidating side-effects occur with time. In Alzheimer's disease, drugs such as acetylcholine esterase inhibitors have also positive effects on the patients' symptoms but their efficacy is mainly observed, at an early stage of the disease.

Consequently, there is a need to find improved treatment for all type of patients affected by a neurodegenerative disorder. Strategies targeting the inhibition of degeneration of neurons are now explored. Agents currently under investigation include in particular calcium channel blockers (isradipine) or antagonists of the receptor of 1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid (compound 1) such as those described in WO2016024246.

Surprisingly and contrary to the state of the art, the Applicant discovered that agonists of the receptor of 1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid (compound 1) demonstrated a neuroprotective effect on neurons.

### SUMMARY

One object of the invention is a composition for use in treating a neurodegenerative disease comprising an agonist of the receptor of 1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid.

In one embodiment, the agonist is selected from compounds of formula I, and triazine dyes; wherein formula I is: 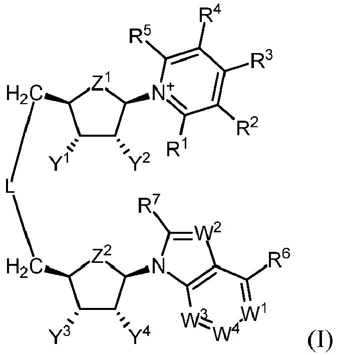 or a pharmaceutically acceptable salt, bioisostere or prodrug thereof, wherein
**Z¹** and **Z²** represent each independently O, S, CH₂ or a halo derivative thereof such as CF₂; preferably **Z¹** and **Z²** represent both O;
**Y¹**, **Y²** and **Y³** represent each independently a group selected from OH, H, NH₂, halo, SH, PO₄²⁻, **OR'** and -OC(O)**R'**, wherein **R'** represents hydrocarbyl; preferably **Y¹**, **Y²** and **Y³** all represent OH;
**Y⁴** represents a group selected from hydroxyl and -OP(O)(O**R"**)(O**R"'**) wherein **R"** and **R"'** represent each independently H, CH₂C(O)CH₃, CH₂OC(O)CH₃, a negative charge; or 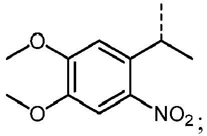 preferably **Y⁴** represents -OP(O)(OH)₂, -OP(O)(O⁻)₂ or -OP(O)(OCH₂OC(O)CH₃)₂;
or **Y³** and **Y⁴** are linked together and **-Y³-Y⁴-** represents -O-P(O)(OH)-O-;
**R¹** represents H;
**R²** represents a group selected from carboxylic acid or carboxylate, aminocarbonyl, hydroxyl, aldehyde, alkyl substituted by a carboxylic acid or a hydroxyl, alkyloxycarbonyl, alkylcarbonyloxyalkyloxycarbonyl; alkylcarbonylalkylcarbonyl, sulfate, phosphino, phosphono, boric acid and 1H-tetrazole; preferably **R²** represents carboxylic acid, carboxylate, or -CO-O-CH₂-O-CO-CH₃;
**R³** represents a group selected from H, alkyl, aryl and amino; preferably **R³** represents H.
**R⁴** represents a group selected from H, alkyl, hydroxyl, amino, carboxylic acid, alkylthio, azido and alkylcarbonylamido, wherein the alkyl group is optionally substituted by a group selected from hydroxyl, amino, azido, alkylcarbonylamido and alkyloxycarbonylamido; or **R⁴** is linked to **R⁵;** preferably **R⁴** represents H;
**R₅** represents H or is linked to **R⁵** so that **-R⁴-R⁵-** represents -CH=CH-CH=CH-;
**W¹**, **W²** and **W³** represent each independently CH or a heteroatom, such as N, P, S or O, preferably **W¹**, **W²** and **W³** all represent N;
**W⁴** represents CR⁸ or N; preferably **W⁴** represents CR⁸;
**R⁶** represents a group selected from amino, hydroxyl, deprotonated hydroxyl and thiol; or when **W¹** is N, **R⁶** is linked to **W¹** by an etheno link; preferably **R⁶** represents amino;
**R⁷** represents a group selected from H, amino, hydroxyl, thiol, halo (preferably Br) and azido; preferably **R⁷** represents H;
**R⁸** represents H; and
L represents a linker group of formula: 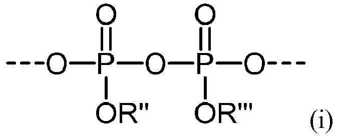 wherein **R"** and **R"'** represent each independently H, CH₂C(O)CH₃, CH₂OC(O)CH₃, a negative charge; or **L** may be selected from one or more of the group comprising: a phosphate, a polyphosphate, a phosphorothioate, a polyethylene glycol, an alkyl, an alkylaryl, a peptide and a polyamine; preferably **L** represents (i).

In another embodiment, the agonist is selected from the group comprising:
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid;
1-((2R,3R,4S,5R)-5-((((((acetoxymethoxy)(((2R,3R,4R,5R)-5-(6-amino-9H-purin-9-yl)-4-((bis(acetoxymethoxy)phosphoryl)oxy)-3-hydroxytetrahydrofuran-2-yl)methoxy)phosphoryl)oxy)(hydroxy)phosphoryl)oxy)methyl)-3,4-dihydroxytetrahydrofuran-2-yl)-3-((acetoxymethoxy)carbonyl)pyridin-1-ium;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-sulfonic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-acetic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]quinoline-1-yl-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-acetamido-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-hydroxy-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-(3-acetamidopropyl)-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-(3-t-Boc-aminopropyl)-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-(3-aminopropyl)-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-(3-hydroxypropyl)-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-(3-azidopropyl)-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-(thiomethyl)-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-4-methyl-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-methyl-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-amino-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-thiomethyl-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-4-amino-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-4-ethyl-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-4-n-butyl-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-4-phenyl-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-carboxy-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-azido-3-carboxylic acid;
1-[(2R,3R,4S,5R)-5-[[[[(2R,3R,4R,5R)-5-(6-amino-8-bromo-purin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid;
1-[(2R,3R,4S,5R)-5-[[[[(2R,3R,4R,5R)-5-(1,N6-etheno-adenine)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid;
1-[(2R,3R,4S,5R)-5-[[[[(2R,3R,4R,5R)-5-(1,N6-etheno-aza-adenine)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid;
1-[(2R,3R,4S,5R)-5-[[[[(2R,3R,4R,5R)-5-(6-amino-8-amino-purin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid;
1-[(2R,3R,4S,5R)-5-[[[[(2R,3R,4R,5R)-5-(6-amino-8-azido-purin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid;
1-[(2R,3R,4S,5R)-5-[[[[(2R,3R,4R,5R)-5-(purin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid;
1-[(2R,3R,4S,5R)-5-[[[[(2R,3R,4R,5R)-5-(6-amino-8-bromo-purin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-azido-3-carboxylic acid;
1-[(2R,3R,4S,5R)-5-[[[[(2R,3R,4R,5R)-5-(hypoxanthine)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxamide;
Reactive Red 120;
Reactive Green 19;
Reactive Green 5;
Cibacron Blue 3GA;
Reactive Yellow 86;
a bioisostere thereof; and/or a prodrug thereof.

In another embodiment, the agonist is selected from the group comprising:
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid; and
1-((2R,3R,4S,5R)-5-((((((acetoxymethoxy)(((2R,3R,4R,5R)-5-(6-amino-9H-purin-9-yl)-4-((bis(acetoxymethoxy)phosphoryl)oxy)-3-hydroxytetrahydrofuran-2-yl)methoxy)phosphoryl)oxy)(hydroxy)phosphoryl)oxy)methyl)-3,4-dihydroxytetrahydrofuran-2-yl)-3-((acetoxymethoxy)carbonyl)pyridin-1-ium.

In another embodiment, the neurodegenerative disease is selected from the group comprising: Parkinson's disease and related disorders, motor neuron diseases, neuro-inflammatory diseases, Alzheimer's disease and related disorders, prion diseases, lysosomal storage diseases, leukodystrophies, Huntington's Disease, Down syndrome, spinal and bulbar muscular atrophy, HIV-Associated Neurocognitive Disorder, Tourette Syndrome, autosomal dominant spinocerebellar ataxia, Friedreich's Ataxia, Dentatorubral pallidoluysian atrophy, myotonic dystrophy (DM1 and DM2), schizophrenia, age associated memory impairment, autism and autism spectrum disorders, attention-deficit hyperactivity disorder, chronic pain, alcohol-induced dementia, progressive non-fluent aphasia, semantic dementia, spastic paraplegia, fibromyalgia, post-Lyme disease, neuropathies, withdrawal symptoms, Alpers' disease, cerebro-oculo-facio-skeletal syndrome, Cockayne syndrome, Leigh's disease, neurodegeneration with brain iron accumulation, opsoclonus myoclonus syndrome, alpha-methylacyl-CoA racemase deficiency, Andermann syndrome, Arts syndrome, Marinesco-Sjögren syndrome, mitochondrial membrane protein-associated neurodegeneration, pantothenate kinase-associated neurodegeneration, polycystic lipomembranous osteodysplasia with sclerosing leukoencephalopathy, riboflavin transporter deficiency neuronopathy, and ataxia telangiectasia.

In another embodiment, the Parkinson's disease and related disorders are selected from the group comprising Parkinson's disease, Parkinson-dementia, autosomal recessive PARK2 and PARK6-linked Parkinsonism, atypical parkinsonian syndromes, such as, for example, progressive supranuclear palsy, corticobasal degeneration syndrome, Lewy bodies dementia, multiple system atrophy, Guadeloupean Parkinsonism and Lytigo-bodig disease.

In another embodiment, the motor neuron diseases are selected from the group comprising amyotrophic lateral sclerosis, frontotemporal dementia, progressive bulbar palsy, pseudobulbar palsy, primary lateral sclerosis, progressive muscular atrophy, spinal muscular atrophy and post-polio syndrome.

In another embodiment, the Alzheimer's disease and related disorders are selected from the group comprising early stage of an Alzheimer's disorder, mild stage of an Alzheimer's disorder, moderate stage of an Alzheimer's disorder, mild to moderate stage of an Alzheimer's disorder, advanced stage of an Alzheimer's disorder, mild cognitive impairment, vascular dementia, mixed dementia, posterior cortical atrophy, Wernicke-Korsakoff Syndrome.

In another embodiment, the neuro-inflammatory diseases are selected from the group comprising Alzheimer's disease, amyotrophic lateral sclerosis, multiple sclerosis, stroke, traumatic brain injury, HIV-associated neurocognitive disorder.

In another embodiment, the prion diseases are selected from the group comprising Creutzfeldt-Jakob disease and its variants, variably protease- sensitive prionopathy, Gerstmann-Sträussler-Scheinker disease and fatal insomnia.

In another embodiment, the lysosomal storage diseases are selected from the group comprising Sialidosis, Galactosialidosis, α-Mannosidosis, β-Mannosidosis, Aspartylglucosaminuria, Fucosidosis, Schindler disease, GM1 gangliosidosis, GM2-gangliosidosis, Tay-Sachs disease, Sandhoff disease, Gaucher disease, Metachromatic leukodystrophy, Multiple sulfatase deficiency, Globoid cell leukodystrophy, Fabry disease, Mucopolysaccharidoses (including without limitations Mucopolysaccharidosis Type I, Type II, Type IIIa, Type IIIb, Type IIIc, Type IIId, Type IV, Type V, Type VI, Type VII, Type VIII, Type IX), Pompe disease, Niemann-Pick disease (including without limitations Type A, Type B and Type C), Farber lipogranulomatosis, Wolman and cholesteryl ester storage disease, Pycnodystostosis, Ceroide lipofuscinoses (including without limitations Ceroide lipofuscinosis 1, 2, 3, 4, 5, 6, 7, and 8), Cystinosis, Salla disease, Danon disease, Chediak-Higashi disease, Griscelli diseases (including without limitations Griscelli disease Type 1, 2 and 3), and Hermansky Pudliak disease.

In another embodiment, the neuropathies are selected from the group comprising peripheral neuropathy and diabetic neuropathy.

In another embodiment, the leukodystrophies are selected from the group comprising X-linked adrenoleukodystrophy, Adrenomyeloneuropathy, Alexander disease

### DEFINITIONS

In the present invention, the following terms have the following meanings:
- **"About":** preceding a figure means plus or less 10% of the value of said figure.
- **"Agonist"** refers to a natural or synthetic compound which binds to the receptor and stimulates the biological activation of the receptor, and thereby the action of the said receptor. It includes any chemical entity that, upon administration to a patient, result in stimulation of a biological activity associated with activation of the receptor in the patient, including any of the downstream biological effects otherwise resulting from the binding to the receptor of its natural ligand. Such agonists include any agent that can stimulate the receptor expression or any of the downstream biological effects of the activation of the receptor.

- **"Bioisostere"** refers to chemical substituents or groups with similar physical or chemical properties which produce broadly similar biological properties to given compound.
- **"Prodrug"** refers to the pharmacologically acceptable derivatives of a compound, such as for example amides or esters, whose *in vivo* biotransformation product generates the biologically active drug. Prodrugs are generally characterized by increased bio-availability and are readily metabolized into biologically active compounds *in vivo.*
- **"Pharmaceutically acceptable excipient"** refers to an excipient that does not produce any adverse, allergic or other unwanted reactions when administered to an animal, preferably a human. It includes any and all solvents, dispersion media, coatings, antibacterial and antifungal agents, isotonic and absorption delaying agents and the like. For human administration, preparations should meet sterility, pyrogenicity, general safety and purity standards as required by regulatory offices, such as, for example, FDA Office or EMA.
- **"Subject"** refers to a mammal, preferably a human. In one embodiment, a subject may be a "patient", i.e. a female or a male, an adult or a child, who/which is awaiting the receipt of, or is receiving medical care or was/is/will be the object of a medical procedure, or is monitored for the development of a disease.
- **"Therapeutically effective amount"** refers to means level or amount of agent that is aimed at, without causing significant negative or adverse side effects to the target, (1) delaying or preventing the onset of a neurodegenerative disease; (2) slowing down or stopping the progression, aggravation, or deterioration of one or more symptoms of a neurodegenerative disease; (3) bringing about ameliorations of the symptoms of a neurodegenerative disease; (4) reducing the severity or incidence of a neurodegenerative disease; or (5) curing a neurodegenerative disease. A therapeutically effective amount may be administered prior to the onset of a neurodegenerative disease, for a prophylactic or preventive action. Alternatively or additionally, the therapeutically effective amount may be administered after the onset of a neurodegenerative disease, for a therapeutic action. In one embodiment, a therapeutically effective amount of the composition is an amount that is effective in reducing at least one symptom of a neurodegenerative disease.
- **"Treating"** or **"treatment"** or **"alleviation"** refers to both therapeutic treatment and prophylactic or preventative measures; wherein the object is to prevent or slow down (lessen) the targeted pathologic condition or disorder. Those in need of treatment include those already with the disorder as well as those prone to have the disorder or those in whom the disorder is to be prevented. A subject or mammal is successfully "treated" for the targeted pathologic disorder if, after receiving a therapeutic amount of the composition according to the methods of the present invention, the patient shows observable effects on one or more of the followings; (i) decrease in neuronal cell death; (ii) decrease in cognitive impairment; (iii) relief to some extent, of one or more of the symptoms associated with the specific disorder or condition; (iv) reduced morbidity and mortality, and improvement in quality of life issues. The above parameters for assessing successful treatment and improvement in the disorder are readily measurable by routine procedures familiar to a physician.
- **"Alkyl"** refers to a hydrocarbyl radical of formula CₙH₂ₙ₊₁ wherein n is a number greater than or equal to 1. Generally, alkyl groups of this invention comprise from 1 to 20 carbon atoms, preferably from 1 to 12 carbon atoms, more preferably from 1 to 6 carbon atoms. Alkyl groups may be linear or branched and may be substituted as indicated herein. Suitable alkyl groups include methyl, ethyl, propyl (n-propyl, i-propyl, n- butyl), butyl (i-butyl, s-butyl and t-butyl), pentyl and its isomers (e.g. n-pentyl, iso-pentyl), and hexyl and its isomers (e.g. n-hexyl, iso-hexyl).
- **"Alkylaryl"** refers to any -aryl-alkyl group.
- **"Alkylcarbonylalkylcarbonyl"** refers to any -C(O)-alkyl-C(O)-alkyl group.
- **"Alkylcarbonylamido"** refers to any -NHC(O)-alkyl group.
- **"Alkylcarbonyloxyalkyloxycarbonyl"** refers to any -C(O)-O-alkyl-O-C(O)-alkyl group.
- **"Alkyloxy"** or **"alkoxy"** refers to any O-alkyl group.
- **"Alkyloxycarbonyl"** refers to any -C(O)O-alkyl group.
- **"Alkyloxycarbonylamido"** refers to any -NHC(O)O-alkyl group.
- **"Alkylthio"** refers to any group -S-alkyl, wherein alkyl is as define above.
- **"Amino"** refers to NH₂ or NH3⁺.
- **"Aminocarbonyl"** refers to the group -CONH₂.
- "**Aryl**" as used herein refers to a polyunsaturated, aromatic hydrocarbyl group having a single ring (i.e. phenyl) or multiple aromatic rings fused together (e.g. naphtyl). or linked covalently, typically containing 5 to 12 atoms; preferably 6 to 10, wherein at least one ring is aromatic. The aromatic ring may optionally include one to two additional rings (either cycloalkyl, heterocyclyl or heteroaryl) fused thereto. Aryl is also intended to include the partially hydrogenated derivatives of the carbocyclic systems enumerated herein. Non-limiting examples of aryl comprise phenyl, biphenylyl, biphenylenyl, 5- or 6-tetralinyl, 1-, 2- , 3-, 4-, 5-, 6-, 7- or 8-azulenyl, naphthalen-1- or -2-yl, A-, 5-, 6 or 7-indenyl, 1- 2-, 3-, 4- or 5-acenaphtylenyl, 3-, 4- or 5-acenaphtenyl, 1-, 2-, 3-, 4- or 10-phenanthryl, 1- or 2- pentalenyl, 4- or 5-indanyl, 5-, 6-, 7- or 8-tetrahydronaphthyl, 1 ,2,3,4-tetrahydronaphthyl, 1 , 4-dihydronaphthyl, 1-, 2-, 3-, 4- or 5-pyrenyl.
- **"Boric acid"** refers to -B(OH)₂.
- **"Carboxylic acid"** refers to -COOH or -COO⁻.
- **"Deprotonated hydroxyl"** refers to the moiety -O⁻.
- **"Etheno link"** refers to -CH=CH- link.
- **"Hydrocarbyl"** refers to a group comprising at least C and H that may optionally comprise one or more other suitable substituents. Examples of such substituents may include halo-, alkoxy-, nitro-, an alkyl group, or a cyclic group. In addition to the possibility of the substituents being a cyclic group, a combination of substituents may form a cyclic group. If the hydrocarbyl group comprises more than one C then those carbons need not necessarily be linked to each other. For example, at least two of the carbons may be linked via a suitable element or group. Thus, the hydrocarbyl group may contain heteroatoms. Suitable heteroatoms will be apparent to those skilled in the art and include, for instance, sulphur, nitrogen, oxygen, silicon and phosphorus. For some embodiments, preferably the hydrocarbyl group is alkyl, alkoxy, alkenyl, alkylene, acyl and alkenylene groups-which may be unbranched-or branched-chain. For some embodiments, preferably the hydrocarbyl group is C₁₋₁₂ alkyl, C₁₋₁₂ alkoxy, C₁₋₁₂ alkenyl, C₁₋₁₂ alkylene, C₁₋₁₂ acyl, and C₁₋₁₂ alkenylene groups-which may be unbranched-or branched-chain. For some embodiments, preferably the hydrocarbyl group is C₁₋₆ alkyl, C₁₋₆ alkoxy, C₁₋₆ alkenyl, C₁₋₆ alkylene, C₁₋₆ acyl, and C₁₋₆ alkenylene groups-which may be unbranched-or branched-chain.
- **"Phosphino"** refers to -PO₂H₂.
- **"Phosphono"** refers to -PO₃H₂.
- **"Phosphorothioate"** as linker refers to -O-P(O)(SH)O-.
- **"Polyphosphate"** as linker refers to -O-[P(O)(OH)O]ₙ- wherein n is an integer preferably ranging from 1 to 20.
- **"Salt"** refers to the acid addition and base salts of compounds of formula I. Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts. Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine, 2-(diethylamino)ethanol, ethanolamine, morpholine, 4- (2- hydroxyethyl)morpholine and zinc salts. Hemisalts of acids and bases may also be formed, for example, hemisulphate and hemicalcium salts. Preferred, pharmaceutically acceptable salts include hydrochloride/chloride, hydrobromide/bromide, bisulphate/sulphate, nitrate, citrate, and acetate. These salts may be prepared by standard procedures, e.g. by reacting a free acid with a suitable organic or inorganic base.

### DETAILED DESCRIPTION

One object of the invention is a composition for treating or for use in treating a neurodegenerative disease comprising an agonist of the receptor of 1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid (compound 1).

Another object of the present invention is also a method for treating a neurodegenerative disease wherein the method comprises, or consists of, or consists essentially of administering to a subject in need thereof, a therapeutically effective amount of an agonist of the receptor of 1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid (compound 1).

According to one embodiment, the agonist used in the present invention is compound 1 or a functional analog thereof. By "functional analog of compound 1" it is meant a compound having biochemical properties similar to those of compound 1. The functional analogs of compound 1 may be endogenous or may be artificially introduced. The functional analog of compound 1 may be structurally close ("structural analog") or structurally distinct from compound 1.

Compound 1 is a Ca²⁺-mobilizing second messenger synthesized in response to extracellular stimuli (Galione A, et al., Sci China Life Sci. 2011 Aug; 54(8)725-732).

Compound 1 specifically and dose-dependently stimulates Ca²⁺ signaling in human T cells. At an activating concentration, compound 1 either evokes repetitive and long-lasting Ca²⁺ oscillations or a single Ca²⁺ spike of high amplitude. Compound 1 can be self-inactivating at high concentrations.

Compound 1 has the following formula:

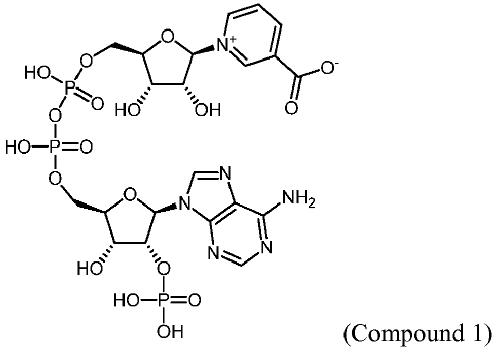

According to one embodiment, the agonist used in the present invention is selected from compound 1, structural analogs of compound 1 and structurally distinct functional analogs of compound 1.

The expression "structural analogs of compound 1" as used herein encompasses at least precursors of compound 1, bioisosteres of compound 1 and prodrugs of compound 1. According to one embodiment, compound 1 and structural analogs thereof are those of formula I: 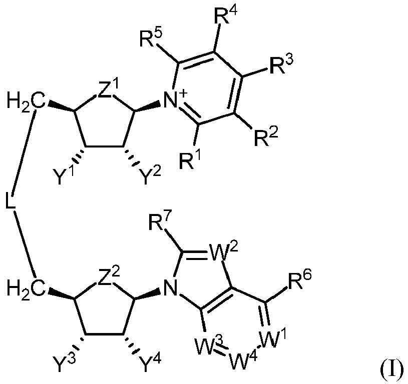 or a pharmaceutically acceptable salt, bioisostere or prodrug thereof, wherein
**Z¹** and **Z²** represent each independently O, S, CH₂ or a halo derivative thereof such as CF₂; preferably **Z¹** and **Z²** represent both O;
**Y¹**, **Y²** and **Y³** represent each independently a group selected from OH, H, NH₂, halo (preferably F), SH, PO₄²⁻, O**R'** and -OC(O)**R'**, wherein **R'** represents hydrocarbyl; preferably **Y¹**, **Y²** and **Y³** all represent OH;

**Y⁴** represents a group selected from hydroxyl and -OP(O)(O**R"**)(O**R"'**) wherein **R"** and
**R"'** represent each independently H, CH₂C(O)CH₃, CH₂OC(O)CH₃, a negative charge; or 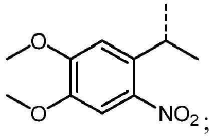 preferably **Y⁴** represents -OP(O)(OH)₂, -OP(O)(O⁻)₂ or -OP(O)(OCH₂OC(O)CH₃)₂;
or **Y³** and **Y⁴** are linked together and **-Y³-Y⁴-** represents -O-P(O)(OH)-O-;
**R¹** represents H;
**R²** represents a group selected from carboxylic acid or carboxylate, aminocarbonyl, hydroxyl, aldehyde, alkyl substituted by a carboxylic acid or a hydroxyl (preferably -CH₂COOH or -CH₂OH), alkyloxycarbonyl (preferably -COOCH₃), alkylcarbonyloxyalkyloxycarbonyl (preferably -CO-O-CH₂-O-CO-CH₃); alkylcarbonylalkylcarbonyl (preferably -COCH₂COCH₃), sulfate, phosphino, phosphono, boric acid (-B(OH)₂) and 1H-tetrazole; preferably **R²** represents carboxylic acid, carboxylate, or -CO-O-CH₂-O-CO-CH₃;
**R³** represents a group selected from H, alkyl (preferably methyl, ethyl or butyl), aryl (preferably phenyl) and amino; preferably **R³** represents H.
**R⁴** represents a group selected from H, alkyl (preferably methyl, ethyl or propyl), hydroxyl, amino, carboxylic acid, alkylthio (preferably -SCH₃), azido and alkylcarbonylamido (preferably acetamido), wherein the alkyl group is optionally substituted by a group selected from hydroxyl, amino, azido, alkylcarbonylamido (preferably acetamido) and alkyloxycarbonylamido (preferably -NHBoc); or **R⁴** is linked to **R⁵;** preferably **R⁴** represents H;
**R₅** represents H or is linked to **R⁵** so that **-R⁴-R⁵-** represents -CH=CH-CH=CH-;
**W¹**, **W²** and **W³** represent each independently CH or a heteroatom, such as N, P, S or O, preferably **W¹**, **W²** and **W³** all represent N;
**W⁴** represents CR⁸ or N; preferably **W⁴** represents CR⁸;
**R⁶** represents a group selected from amino, hydroxyl, deprotonated hydroxyl and thiol;
or when **W¹** is N, **R⁶** is linked to **W¹** by an etheno link; preferably **R⁶** represents amino;
**R⁷** represents a group selected from H, amino, hydroxyl, thiol, halo (preferably Br) and azido; preferably **R⁷** represents H;
**R⁸** represents H; and
**L** represents a linker group of formula: 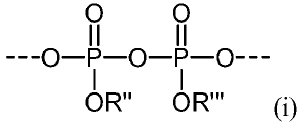 wherein **R"** and **R"'** represent each independently H, CH₂C(O)CH₃, CH₂OC(O)CH₃, a negative charge; or **L** may be selected from one or more of the group comprising: a phosphate, a polyphosphate, a phosphorothioate, a polyethylene glycol, an alkyl, an alkylaryl, a peptide and a polyamine; preferably **L** represents (i).

According to one embodiment, **L** represents a linker of formula (i) selected from:

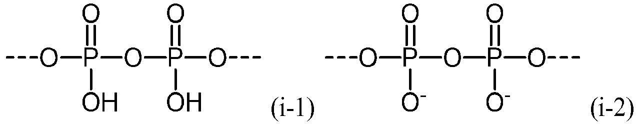

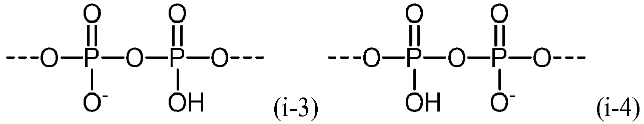

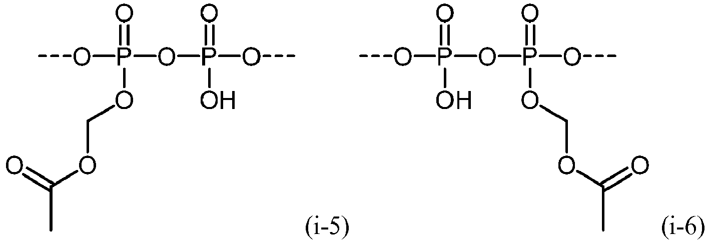

Preferably, L represents a linker of formula (i-1) or (i-6).

Compound 1 is charged and cannot cross cell membranes. Therefore, an inactive, lipophilic ester precursor acetoxymethyl ester of compound 1 (hereafter referred to as compound 2) has been synthesized which crosses membranes and rapidly regenerates compound 1 in the cytosol following the action of endogenous esterases.

Therefore, in one embodiment of the invention, the structural analog of compound 1 is compound 2, which has the following formula: 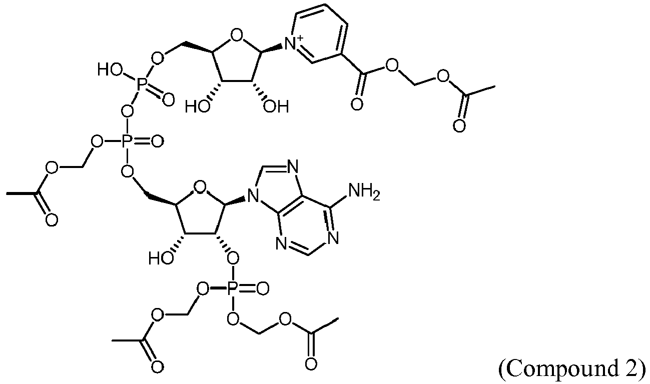 corresponding to 1-((2R,3R,4S,5R)-5-((((((acetoxymethoxy)(((2R,3R,4R,5R)-5-(6-amino-9H-purin-9-yl)-4-((bis(acetoxymethoxy)phosphoryl)oxy)-3-hydroxytetrahydrofuran-2-yl)methoxy)phosphoryl)oxy)(hydroxy)phosphoryl)oxy)methyl)-3,4-dihydroxytetrahydrofuran-2-yl)-3-((acetoxymethoxy)carbonyl)pyridin-1-ium.

In one embodiment, the agonist used in the present invention is compound 1 or compound 2.

In one embodiment, the structural analog of compound 1 used in the invention is described in the patent application WO02/11736 incorporated herein by reference and is of following formula II: 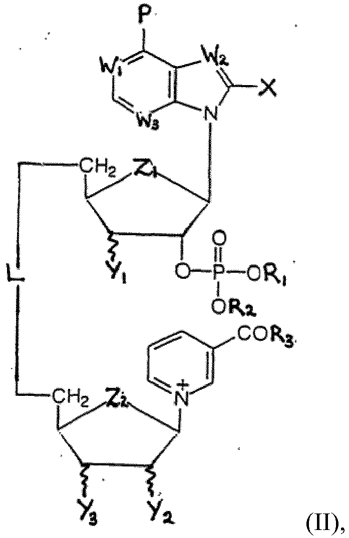
wherein P is a substituent group independently selected from NH₂, OH, SH;
each of W₁, W₂ or W₃ is independently selected from either a CH or a heteroatom, such as N, P, S or O, preferably N;
X is a substituent group independently selected from OH, SH, NH₂, or a halo group (preferably Br);
each of R₁, R₂ or R₃ is independently selected from H or -CH₂-C(O)-CH₃;
each of Y₁, Y₂ or Y₃ is independently selected from OH, H, NH₂, halo (preferably F), SH, OR₄, or -OC(O)R₄ wherein R⁴ is a hydrocarbyl;
each of Z₁ or Z₂ is independently selected from O, S, CH₂ or a halo derivative thereof, preferably CF₂;
and L is a linker group, suitably the linker group may have the formula: 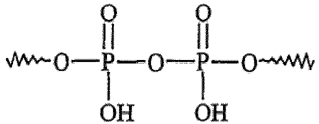
or may be selected from one or more of the group comprising: a phosphate, a polyphosphate, a phosphorothioate, a polyethylene glycol, an alkyl, an alkylaryl, a peptide and a polyamine; or isomeric forms of the compound of Formula (II).

Structural analogs of compound 1 are also described in Lee HC and Aarhus RA J Biol Chem. 1997 Aug 15;272(33):20378-83; Billington RA et al. Cell Calcium. 2005 January; 37(1):81-6; Jain P et al. J Med Chem. 2010 November 11, 53(21): 7599-7612; Ali RA et al. Cell Calcium. 2014 February; 55(2): 93-103; Trabbic CJ et al. J Med Chem. 2015 April; 58(8): 3593-3610 which are all incorporated herein by reference.

In one embodiment of the invention, the structural analog of compound 1 is modified on the pyridine base. The presence of a substituent of a negative charge at the 3-position of the pyridine ring is of importance for the activity towards the receptor.

Examples of modifications on the pyridine base of compound 1 include, without limitation, the following substitutions: pyridine-3-sulfonic acid; pyridine-3-acetic acid; quinoline 3-carboxylic acid; 5-acetamido-pyridine; 5-hydroxy-pyridine; 5-(3-acetamidopropyl)-pyridine; 5-(3-t-Boc-aminopropyl)-pyridine; 5-(3-aminopropyl)-pyridine; 5-(3-hydroxypropyl)-pyridine; 5-(3-azidopropyl)-pyridine; 5-(thiomethyl)-pyridine; 4-methyl-pyridine; 5-methyl-pyridine; 5-amino-pyridine; 5-thiomethyl-pyridine; 4-amino-pyridine; 4-ethyl-pyridine; 4-n-butyl-pyridine; 4-phenyl-pyridine; 5-ethyl-pyridine; 5-carboxy-pyridine; 5-azido-pyridine.

In another embodiment of the invention, the structural analog of compound 1 is modified on adenine base.

Examples of modifications on adenine of compound 1 include, without limitation, the following moieties: 1,N⁶-etheno-adenine; 1,N⁶-etheno-aza-adenine; 8-amino-adenine; 8-azido-adenine; deamino-adenine; 8-bromo-adenine.

According to one embodiment, the structural analog of compound 1 is modified on the pyridine base and on the adenine base. Modifications listed above also apply in this embodiment. Examples of modifications on pyridine and on adenine include, without limitation, the following modifications: 5-azido-pyridine and 8-bromo-adenine; 3-amide-pyridine and hypoxanthine.

According to another embodimentfcomp, the agonist used in the present invention is a structurally distinct functional analog of compound 1.

In one embodiment, structurally distinct functional analogs of compound 1 are triazine dyes such as those described in R.A. Billington, et al., Br. J. Pharmacol., 142 (2004), pp. 1241-1246, which is incorporated herein by reference. Especially, triazine dyes are for example Reactive Red 120 (RR120), Reactive Green 19 (RG19), Reactive Green 5 (RG5), Cibacron Blue 3GA and Reactive Yellow 86, among which Reactive Red 120 (RR120) is particularly preferred.

Triazine dyes Reactive Red 120 (RR120), Reactive Green 19 (RG19), Reactive Green 5 (RG5), Cibacron Blue 3GA and Reactive Yellow 86 may be obtained from Sigma-Aldrich supplier.

Thus, in one embodiment, the agonist used in the present invention is selected from compound 1, structural analogs of formula I and triazine dyes.

Another object of the invention is a pharmaceutical composition for treating or for use in treating a neurodegenerative disease comprising, or consisting of, or consisting essentially of an agonist of the receptor of 1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid (compound 1) as described here above and at least one pharmaceutically acceptable excipient.

Another object of the invention is a medicament for treating or for use in treating a neurodegenerative disease comprising, or consisting of, or consisting essentially of an agonist of the receptor of 1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid (compound 1) as described here above.

Another object of the invention is a pharmaceutical composition for treating or for use in treating a neurodegenerative disease comprising, or consisting of, or consisting essentially of compound 1 and/or a functional analog thereof and/or a structural analog thereof described here above and at least one pharmaceutically acceptable excipient.

Another object of the invention is a medicament for treating or for use in treating a neurodegenerative disease comprising, or consisting of, or consisting essentially of compound 1 and/or a functional analog thereof and/or a structural analog thereof described here above.

As used herein, the term "consisting essentially of", with reference to a compound, a composition, pharmaceutical composition or medicament, means that the at least one compound of the invention is the only one therapeutic agent or agent with a biologic activity within said composition, pharmaceutical composition or medicament.

Pharmaceutically acceptable excipients include water, saline, Ringer's solution, dextrose solution, and solutions of ethanol, glucose, sucrose, dextran, mannose, mannitol, sorbitol, polyethylene glycol (PEG), phosphate, acetate, gelatin, collagen, Carbopol®, vegetable oils, and the like. One may additionally include suitable preservatives, stabilizers, antioxidants, antimicrobials, and buffering agents, such as, for example, BHA, BHT, citric acid, ascorbic acid, tetracycline, and the like.

Other examples of pharmaceutically acceptable excipients that may be used in the composition of the invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene- block polymers, polyethylene glycol and wool fat.

In addition, pharmaceutically acceptable excipients may comprise some excipients, such as, for example, surfactants (e.g. hydroxypropylcellulose); suitable carriers, such as, for example, solvents and dispersion media containing, for example, water, ethanol, polyol (e.g. glycerol, propylene glycol, and liquid polyethylene glycol, and the like), suitable mixtures thereof, and vegetable oils, such as, for example, peanut oil and sesame oil; isotonic agents, such as, for example, sugars or sodium chloride; coating agents, such as, for example, lecithin; agents delaying absorption, such as, for example, aluminum monostearate and gelatin; preservatives, such as, for example, benzalkonium chloride, benzethonium chloride, chlorobutanol, thimerosal and the like; buffers, such as, for example, boric acid, sodium and potassium bicarbonate, sodium and potassium borates, sodium and potassium carbonate, sodium acetate, sodium biphosphate and the like; tonicity agents, such as, for example, dextrose, potassium chloride, propylene glycol, sodium chloride; antioxidants and stabilizers, such as, for example, sodium bisulfite, sodium metabisulfite, sodium thiosulfite, thiourea and the like; nonionic wetting or clarifying agents, such as, for example, polysorbate 80, polysorbate 20, poloxamer 282 and tyloxapol; viscosity modifying agents, such as, for example dextran 40, dextran 70, gelatin, glycerin, hydroxyethylcellulose, hydroxmethylpropylcellulose, lanolin, methylcellulose, petrolatum, polyethylene glycol, polyvinyl alcohol, polyvinylpyrrolidone, carboxymethylcellulose; and the like.

Examples of neurodegenerative diseases, include without limitation, either sporadic or inherited forms of neurodegenerative diseases, Parkinson's disease and related disorders (including without limitation inherited forms of Parkinson's disease, Parkinson-dementia, atypical parkinsonian syndromes, such as, for example, progressive supranuclear palsy, corticobasal degeneration syndrome, Lewy body dementia, multiple system atrophy, Guadeloupean Parkinsonism or Guam syndrome), Alzheimer's disease and related disorders (including without limitation, early stage of an Alzheimer's disorder, mild stage of an Alzheimer's disorder, moderate stage of an Alzheimer's disorder, mild to moderate stage of an Alzheimer's disorder, advanced stage of an Alzheimer's disorder, mild cognitive impairment, vascular dementia, mixed dementia, posterior cortical atrophy, Wernicke-Korsakoff Syndrome), neuro-inflammatory diseases, motor neuron diseases (including without limitation, amyotrophic lateral sclerosis, frontotemporal dementia (also referred as frontotemporal lobar degeneration or Picks disease), progressive bulbar palsy, pseudobulbar palsy, primary lateral sclerosis, progressive muscular atrophy, spinal muscular atrophy and post-polio syndrome), prion diseases (including without limitations, Creutzfeldt-Jakob disease and its variants, variably protease-sensitive prionopathy, Gerstmann-Sträussler-Scheinker disease and fatal insomnia), lysosomal storage diseases (including without limitations, Sialidosis, Galactosialidosis, α-Mannosidosis, β-Mannosidosis, Aspartylglucosaminuria, Fucosidosis, Schindler disease, GM1 gangliosidosis, GM2-gangliosidosis, Tay-Sachs disease, Sandhoff disease, Gaucher disease, Metachromatic leukodystrophy, Multiple sulfatase deficiency, Globoid cell leukodystrophy, Fabry disease, Mucopolysaccharidoses (including without limitations Mucopolysaccharidosis Type I, Type II, Type IIIa, Type IIIb, Type IIIc, Type IIId, Type IV, Type V, Type VI, Type VII, Type VIII, Type IX), Pompe disease, Niemann-Pick disease (including without limitations Type A, Type B and Type C), Farber lipogranulomatosis, Wolman and cholesteryl ester storage disease, Pycnodystostosis, Ceroide lipofuscinoses (including without limitations Ceroide lipofuscinosis 1, 2, 3, 4, 5, 6, 7, and 8), Cystinosis, Salla disease, Danon disease, Chediak-Higashi disease, Griscelli diseases (including without limitations Griscelli disease Type 1, 2 and 3), and Hermansky Pudliak disease, leukodystrophies (including without limitations X-linked adrenoleukodystrophy, Adrenomyeloneuropathy Alexander disease), multiple sclerosis, Huntington's Disease (HD), Down syndrome, spinal and bulbar muscular atrophy (Kennedy's disease), HIV-Associated Neurocognitive Disorder, Tourette Syndrome, autosomal dominant spinocerebellar ataxia, Friedreich's Ataxia, Dentatorubral pallidoluysian atrophy (DRPLA/Haw-River syndrome), myotonic dystrophy (DM1 and DM2), schizophrenia, age associated memory impairment, autism and autism spectrum disorders, attention-deficit hyperactivity disorder, chronic pain, stroke, traumatic brain injury, spinal cord injury, alcohol-induced dementia, progressive non-fluent aphasia, semantic dementia, spastic paraplegia, fibromyalgia, post-Lyme disease, neuropathies (including without limitations peripheral neuropathy and diabetic neuropathy), withdrawal symptoms, Alpers' disease, cerebro-oculo-facio-skeletal syndrome, Cockayne syndrome, Leigh's disease, neurodegeneration with brain iron accumulation, opsoclonus myoclonus syndrome, alpha-methylacyl-CoA racemase deficiency, Andermann syndrome, Arts syndrome, Marinesco-Sjögren syndrome, mitochondrial membrane protein-associated neurodegeneration, pantothenate kinase-associated neurodegeneration, polycystic lipomembranous osteodysplasia with sclerosing leukoencephalopathy, riboflavin transporter deficiency neuronopathy, ataxia telangiectasia.

Related disorders of Parkinson's disease include without limitation, Parkinson's disease, Parkinson-dementia, autosomal recessive PARK2 and PARK6-linked Parkinsonism, atypical parkinsonian syndromes, such as, for example, progressive supranuclear palsy, corticobasal degeneration syndrome, Lewy bodies dementia, multiple system atrophy, Guadeloupean Parkinsonism and Lytigo-bodig disease (also named Guam syndrome).

Related disorders of Alzheimer's disease include without limitation, early stage of an Alzheimer's disorder, mild stage of an Alzheimer's disorder, moderate stage of an Alzheimer's disorder, mild to moderate stage of an Alzheimer's disorder, advanced stage of an Alzheimer's disorder, mild cognitive impairment, vascular dementia, mixed dementia, posterior cortical atrophy, Wernicke-Korsakoff Syndrome.

Neuro-inflammatory diseases include without limitation, Alzheimer's disease, amyotrophic lateral sclerosis, multiple sclerosis, stroke, traumatic brain injury, HIV-associated neurocognitive disorder.

Motor neuron diseases include without limitation, amyotrophic lateral sclerosis, frontotemporal dementia (also referred as frontotemporal lobar degeneration or Picks disease), progressive bulbar palsy, pseudobulbar palsy, primary lateral sclerosis, progressive muscular atrophy, spinal muscular atrophy and post-polio syndrome.

Prion diseases include without limitation, Creutzfeldt-Jakob disease and its variants, variably protease- sensitive prionopathy, Gerstmann-Sträussler-Scheinker disease and fatal insomnia.

Lysosomal storage diseases include without limitation, Sialidosis, Galactosialidosis, α-Mannosidosis, β-Mannosidosis, Aspartylglucosaminuria, Fucosidosis, Schindler disease, GM1 gangliosidosis, GM2-gangliosidosis, Tay-Sachs disease, Sandhoff disease, Gaucher disease, Metachromatic leukodystrophy, Multiple sulfatase deficiency, Globoid cell leukodystrophy, Fabry disease, Mucopolysaccharidoses (including without limitations Mucopolysaccharidosis Type I, Type II, Type IIIa, Type IIIb, Type IIIc, Type IIId, Type IV, Type V, Type VI, Type VII, Type VIII, Type IX), Pompe disease, Niemann-Pick disease (including without limitations Type A, Type B and Type C), Farber lipogranulomatosis, Wolman and cholesteryl ester storage disease, Pycnodystostosis, Ceroide lipofuscinoses (including without limitations Ceroide lipofuscinosis 1, 2, 3, 4, 5, 6, 7, and 8), Cystinosis, Salla disease, Danon disease, Chediak-Higashi disease, Griscelli diseases (including without limitations Griscelli disease Type 1, 2 and 3), and Hermansky Pudliak disease.

Leukodystrophies include without limitation, X-linked adrenoleukodystrophy, Adrenomyeloneuropathy, Alexander disease.

Neuropathies include without limitation, peripheral neuropathy and diabetic neuropathy.

In one embodiment, compound 1 or a functional analog thereof or a structural analog thereof as described here above or the pharmaceutical composition or the medicament of the invention is to be administered at a dose determined by the skilled artisan and personally adapted to each subject.

In one embodiment of the invention, compound 1 or a functional analog thereof or a structural analog thereof as described here above, the pharmaceutical composition, the medicament of the invention is to be administered at a therapeutically effective amount.

It will be understood that the total daily usage of the compound of the invention, composition, pharmaceutical composition and medicament of the invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific composition employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from about 1 to about 10000 mg per adult per day, preferably 2 to about 2000, more preferably from about 5 to about 500 mg per adult per day. Preferably, the compositions contain 1, 10, 20, 30, 40, 50, 60, 70, 80, 90, 100, 250, 500, 1000 and 2000 mg of the active ingredient for the symptomatic adjustment of the dosage to the patient to be treated. A medicament may typically contain from about 1 to about 10000 mg of the active principle, preferably about 2 to about 2000, more preferably from about 5 to about 500 mg of the active ingredient. An effective amount of the drug may ordinarily be supplied at a dosage level from about 0.01 mg/kg to about 100 mg/kg of body weight per day, preferably from about 0.02 mg/kg to 20 mg/kg of body weight per day, more preferably from about 0.05 mg/kg to 5 mg/kg of body weight per day.

In one embodiment, compound 1 or a functional analog thereof or a structural analog thereof as described here above, the pharmaceutical composition or the medicament of the invention is to be administered systemically or locally.

In one embodiment, compound 1 or a functional analog thereof or a structural analog thereof as described here above, the pharmaceutical composition or the medicament of the invention is to be administered orally, by injection, topically, nasally, by inhalation, buccally, sublingually, rectally, intratracheally, by endoscopy, transmucosally, by percutaneous administration or by perispinal administration.

In one embodiment, compound 1 or a functional analog thereof or a structural analog thereof as described here above, the pharmaceutical composition, the medicament of the invention is to be administered by injection, preferably is to be systemically injected. Examples of formulations adapted to systemic injections include, but are not limited to: liquid solutions or suspensions, solid forms suitable for solution in, or suspension in, liquid prior to injection. Examples of systemic injections include, but are not limited to, intravenous, subcutaneous, intramuscular, intradermal, and intraperitoneal injection, or perfusion. In another embodiment, when injected, the composition, the pharmaceutical composition or the medicament of the invention is sterile. Methods for obtaining a sterile pharmaceutical composition include, but are not limited to, GMP synthesis (GMP stands for "Good manufacturing practice").

In one embodiment, compound 1 or a functional analog thereof or a structural analog thereof as described here above, the pharmaceutical composition, the medicament of the invention is to be orally administered. Examples of formulations adapted to oral administration include, but are not limited to: solid forms, liquid forms and gels. Examples of solid forms adapted to oral administration include, but are not limited to, pill, tablet, capsule, soft gelatine capsule, hard gelatine capsule, caplet, compressed tablet, cachet, wafer, sugar-coated pill, sugar coated tablet, or dispersing/or disintegrating tablet, powder, solid forms suitable for solution in, or suspension in, liquid prior to oral administration and effervescent tablet. Examples of liquid forms adapted to oral administration include, but are not limited to, solutions, suspensions, drinkable solutions, elixirs, sealed phial, potion, drench, syrup and liquor.

In another embodiment, compound 1 or a functional analog thereof or a structural analog thereof as described here above, the pharmaceutical composition, the medicament of the invention is to be topically administered. Examples of formulations adapted to topical administration include, but are not limited to, sticks, waxes, creams, lotions, ointments, balms, gels, masks, leave-on washes and/or the like.

In one embodiment, compound 1 or a functional analog thereof or a structural analog thereof as described here above, the pharmaceutical composition or the medicament of the invention is to be administered in a sustained-release form. In another embodiment, the composition, the pharmaceutical composition or the medicament of the invention comprises a delivery system that controls the release of the agent.

In one embodiment, compound 1 or a functional analog thereof or a structural analog thereof as described here above, the pharmaceutical composition or the medicament of the invention is to be administered perispinally or intra-nasally in improved delivery, either by local diffusion; by improved transport into the cerebrospinal fluid (CSF); or by direct transport into the CNS.

In one embodiment, a therapeutically effective amount of compound 1 or a functional analog thereof or a structural analog thereof as described here above, the pharmaceutical composition or the medicament of the invention is administered at least once a day, twice a day, or at least three times a day.

In another embodiment, a therapeutically effective amount of compound 1 or a functional analog thereof or a structural analog thereof as described here above, the pharmaceutical composition, medicament of the invention is administered every two, three, four, five, or six days.

In another embodiment, a therapeutically effective amount of compound 1 or a functional analog thereof or a structural analog thereof as described here above, the pharmaceutical composition, medicament of the invention is administered twice a week, every week, every two weeks, or once a month.

In another embodiment, a therapeutically effective amount of compound 1 or a functional analog thereof or a structural analog thereof as described here above or the pharmaceutical composition of the invention, the medicament of the invention is administered every month for a period at least 2; 3; 4; 5; 6 months or for the rest of the life of the subject.

In another embodiment, a therapeutically effective amount of compound 1 or a functional analog thereof or a structural analog thereof as described here above or the pharmaceutical composition or the medicament of the invention of the invention ranges from about 1 µg to 1000 g, 1 mg to 100g, 10 mg to 1 g.

In another embodiment, a therapeutically effective amount of compound 1 or a functional analog thereof or a structural analog thereof as described here above, the pharmaceutical composition or the medicament of the invention ranges from about 0.1 µg/kg to 1 g/kg of body weight, 0.1 mg/kg of body weight to 0.1 g/kg, 1.5 mg/kg to 0.01 g/kg of body weight.

In one embodiment, the subject is affected, preferably is diagnosed with a neurodegenerative disease. In another embodiment, the subject of the invention is at risk of developing a neurodegenerative disease.

In one embodiment of the invention, the subject has a familial predisposition to a neurodegenerative disease. In another embodiment of the invention, the subject has a genetic predisposition to a neurodegenerative disease.

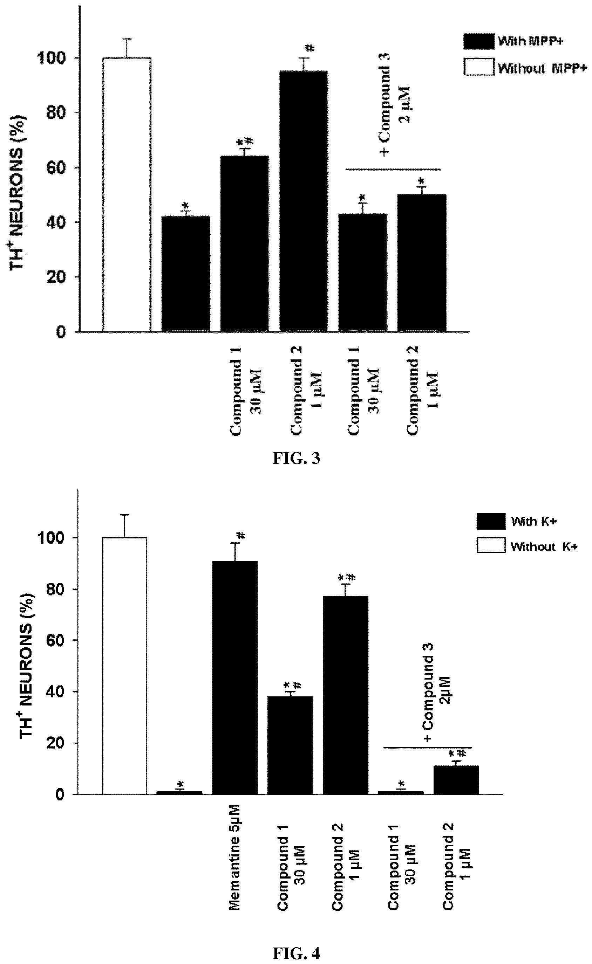

Examples of genetic predisposition linked to a neurodegenerative disease include without limitation, tau protein gene; SHC2; HTT gene; SOD1, ALS2, SETX, SPG11, FUS, VAPB, ANG, TARDBP, FIG4, OPTN, ATXN2, VCP, UBQLN2, SIGMAR1, CHMP2B, PFN1, ERBB4, HNRNPA1, MATR3, TUBA4A, C9orf72, CHCHD10, SQSTM1, TBK1, FXN, DR15, DQ6, HLA-C554, HLA-DRB1*11; SCA1-SCA3, SCA8, SCA10, SCA12, SCA13, SCA14, SCA15/SCA16, SCA17, SCA27- SCA28, SCA35, SCA36, DRPLA; DMPK; ZNF9; MAPT; TDP43, GRN, genes in the serotoninergic, dopaminergic and catecholaminergic systems.

Examples of genetic predisposition linked to Parkinson's disease include without limitation, α-synuclein, leucine-rich repeat kinase 2 (LRRK-2), glucocerebrosidase (GBA), PARK6, PINK1. In one embodiment, the genetic predisposition is an autosomal recessive mutation.

Examples of genetic predisposition linked to Alzheimer's disorder include without limitation, the ε4 allele of the apolipoprotein E (APOE); protein Tau, TRPM2 (clusterin); cytochrome P46; oxidized low density lipoprotein receptor 1; angiotensin 1-converting enzyme; PICALM (phosphatidylinositol-binding clathrin assembly protein), Myc box-dependent-interacting protein 1 (BIN1); CD2-associated protein (CD2AP); CR1 (complement receptor 1); CD33; ephrin type-A receptor 1 (EPHA1), membrane-spanning 4-domains, subfamily A, member 4A and 6A (MS4A4A/MS4A6A); ATP-binding cassette, sub-family A1 (ABC1), ABCA7; DSG2; PTK2B; SORL1; SLC24A4; RIN3; CASS4; INPP5D; NME8; MEF2C; FERMT2; ZCWPW1; TREML2; SPPL2A; ECHDC3; NDUFAF6; TRIP4; ADAMST20; SCIMP; HS3ST1; CLU; HLA-DRB5-HLA-DRB1; and CELF1.

In one embodiment of the invention, the subject has an autosomal dominant familial form of Alzheimer disease. Examples of mutations for autosomal dominant familial form of Alzheimer disease are but not limited to, mutations within amyloid precursor protein (APP), presenilins 1 and 2.

Another object of the present invention is a method for decreasing oxidative stress in a subject in need thereof comprising administering a therapeutically effective amount of compound 1 or a functional analog thereof or a structural analog thereof.

Another object of the present invention is a method for promoting neurons survival in a subject in need thereof comprising administering a therapeutically effective amount of compound 1 or a functional analog thereof or a structural analog thereof.

Methods to detect neuron survival are well known to the skilled artisan and include without limitation, magnetic resonance imaging (MRI), Positron Emission Tomography.

Another object of the present invention is a method for decreasing inflammation in a subject in need thereof comprising administering a therapeutically effective amount of compound 1 or a functional analog thereof or a structural analog thereof.

In one embodiment, the method decreases neuro-inflammation.

Methods to detect neuro-inflammation are well known to the skilled artisan and include without limitation collect of cerebrospinal fluid and analyses.

Another object of the present invention is a method for decreasing excitotoxicity in a subject in need thereof comprising administering a therapeutically effective amount of compound 1 or a functional analog thereof or a structural analog thereof.

Methods to detect excitotoxicity are well known to the skilled artisan and include without limitation two-dimensional H-magnetic resonance spectroscopy (MRS), MRI, Manganese-enhanced MRI.

Another object of the present invention is a method for decreasing neurotrophic factor deprivation in a subject in need thereof comprising administering a therapeutically effective amount of compound 1 or a functional analog thereof or a structural analog thereof.

Methods to detect neurotrophic factor deprivation are well known to the skilled artisan and include without limitation two-dimensional H-magnetic resonance spectroscopy (MRS).

Another object of the present invention is a method for decreasing mitochondrial dysfunction in a subject in need thereof comprising administering a therapeutically effective amount of compound 1 or a functional analog thereof or a structural analog thereof.

Methods to detect mitochondrial dysfunction are well known to the skilled artisan and include without limitation magnetic resonance spectroscopy, two-dimensional H-magnetic resonance spectroscopy (MRS), optical spectroscopy.

### BRIEF DESCRIPTION OF THE DRAWINGS

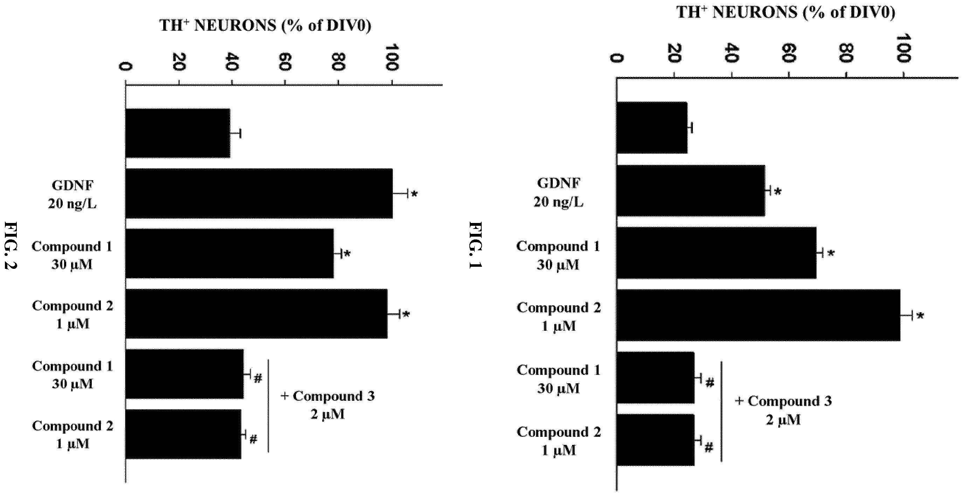

**Figure 1** is a bargraph showing the neuroprotective effect of compound 1 and compound 2 for dopaminergic (DA) neurons in a model of midbrain cultures where these neurons degenerate spontaneously, selectively and progressively as they mature. Number of DA (TH⁺) neurons in 10-day in vitro (DIV) cultures chronically exposed to compound 1 or compound 2 in the presence or absence of (1R,3S)-1-[3-[[4-(2-Fluorophenyl)piperazin-1-yl]methyl]-4-methoxyphenyl]-2,3,4,9-tetrahydro-1H-pyrido[3,4-b]indole-3-carboxylic acid (compound 3) (2 µM). GDNF was used as positive control at a concentration of 20 ng/ml. Results are expressed in % of TH⁺ neurons at DIV0, i.e., after plating. Each bar is the mean value of 3 independent experiments * P <0.05 vs untreated cultures; # P <0.05 vs cultures treated with the corresponding treatment in the absence of compound 3.
**Figure 2** is a bargraph showing the neuroprotective effect of compound 1 and compound 2 for DA (TH⁺) neurons in a culture model where these neurons degenerate following GDNF withdrawal. Impact of a treatment with compound 1 (30 µM) or compound 2 (1 µM) in the presence or not of compound 3 (2 µM) in cultures initially exposed to 20 ng/ml GDNF for 10 DIV and then deprived of the peptide between 11 and 15 DIV. GDNF was used as positive control at a concentration of 20 ng/ml. Results are expressed in % of neurons at DIV10. Each bar is the mean value of 3 independent experiments. * P <0.05 vs untreated cultures; # P <0.05 vs cultures treated with the corresponding treatment in the absence of compound 3.
**Figure 3** is a bargraph showing the neuroprotective effect of compound 1 and compound 2 against MPP⁺-induced DA cell death. DA (TH⁺) cell survival in midbrain cultures treated with MPP⁺ (3 µM) between 5 and 7 DIV exposed or not to compound 1 (30 µM) or compound 2 (1 µM) in the presence or absence of compound 3 (2 µM). Results are expressed in % of corresponding control cultures. Each bar is the mean value of 3 independent experiments. * P <0.05 vs without MPP⁺; # P <0.05 vs with MPP⁺.
**Figure 4** is a bargraph showing the neuroprotective effect of compound 1 and compound 2 against excitotoxicity triggered by a prolonged treatment of midbrain cultures with elevated K⁺ (30 mM). DA (TH⁺) cell survival in midbrain cultures treated with K⁺ (30 mM) between 5 and 7 DIV and exposed to compound 1 (30 µM) or compound 2 (1 µM) in the presence or absence of compound 3 (2 µM). Memantine (5 µM), a blocker of NMDA glutamate receptors was used as reference protective molecule. Results are expressed in % of corresponding control cultures. Each bar is the mean value of 3 independent experiments. * P <0.05 vs without added K⁺; # P <0.05 vs with elevated K⁺.

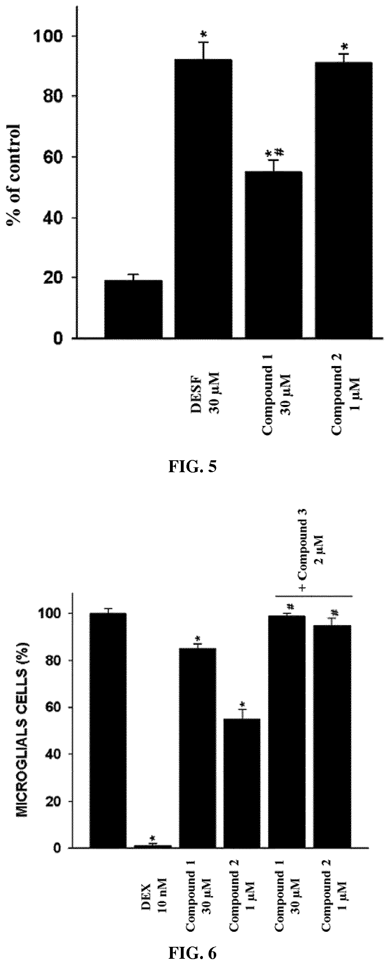

**Figure 5** is a bargraph showing the neuroprotective effect of compound 1 and compound 2 against oxidative stress. Number of DA (TH⁺) neurons in 10-DIV cultures chronically exposed to compound 1 (30 µM) or compound 2 (1 µM). The iron chelator desferrioxamine (DESF) was used as reference protective molecule at a concentration of 10 µM. Results are expressed in % of corresponding control cultures. Each bar is the mean value of 3 independent experiments. * P <0.05 vs untreated cultures; # P <0.05 vs DESF.
**Figure 6** is a bargraph showing the effect of compound 1 and compound 2 on microglial cell numbers. Number of CD11b⁺ microglial cells in 10-DIV midbrain cultures chronically exposed to compound 1 (30 µM) or compound 2 (1 µM) in the presence or absence of the compound 1 antagonist, compound 3 (2 µM). The steroid drug dexamethasone (DEX) was used as positive control, at a concentration of 10 nM. Results are expressed in % of corresponding control cultures. Each bar is the mean value of 3 independent experiments. * P <0.05 vs untreated cultures; # P <0.05 vs cultures treated with the corresponding treatment in the absence of compound 3.

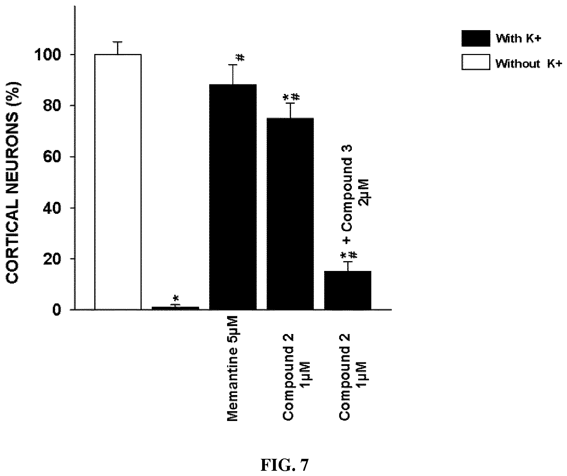

**Figure 7** is a bargraph showing the neuroprotective effect of compound 2 against excitotoxicity triggered by a prolonged treatment of cortical cultures with elevated K⁺ (30 mM). Number of Microtubule-Associated Protein 2 (MAP2) positive neurons in cortical cultures treated with K⁺ (30 mM) between 5 and 7 DIV and exposed to compound 2 (1 µM) in the presence or absence of compound 3 (2 µM). Memantine (5 µM), a blocker of NMDA glutamate receptors was used as reference protective molecule. Results are expressed in % of corresponding control cultures. Each bar is the mean value of 3 independent experiments. * P <0.05 vs without added K⁺; # P <0.05 vs with elevated K⁺.

### EXAMPLES

The present invention is further illustrated by the following examples.

### Example 1: Neuroprotective effect of compound 1 and compound 2

### Prevention of spontaneous and progressive dopaminergic (DA) cell death in midbrain cultures.

We report here that compound 1 and a structural analog of compound 1, compound 2, concentration-dependently increased the number of midbrain DA (TH⁺) neurons in culture conditions where these neurons die spontaneously, selectively and progressively as they mature. In this particular model, the optimal neuroprotective concentrations for compound 1 and compound 2 were 30 µM and 1 µM, respectively after chronic treatments for 10 days **(****Figure 1****).** Interestingly, compound 1 and compound 2, both conferred better neuroprotection than GDNF (20 ng/ml), a highly potent trophic peptide for DA neurons. Noteworthy, the neuroprotective effect of compound 1 and compound 2 were fully antagonized in the presence of (1R,3S)-1-[3-[[4-(2-Fluorophenyl)piperazin-1-yl]methyl]-4-methoxyphenyl]-2,3,4,9-tetrahydro-1H-pyrido[3,4-b]indole-3-carboxylic acid (compound 3) (2 µM), an antagonist of compound 1.

### Protection of DA neurons that degenerate after GDNF deprivation.

To determine whether the protective effect of compound 1 and compound 2 remained observable in more mature DA neurons, we used midbrain cultures in which the spontaneous death process was postponed for 10 days by chronic application of GDNF (20 ng/ml). Ablation of GDNF from these cultures at 11 DIV led to a massive and selective loss of TH⁺ neurons within the next 5 days in agreement with previous data (Guerreiro et al., Mol Pharmacol. 2008 Oct;74(4):980-9). Interestingly, a large population of these neurons were saved from death if compound 1 (30 µM) was added to the culture medium in replacement of GDNF **(****Figure 2****),** and totally if compound 2 (1 µM) was added instead of compound 1. This effect was abolished when the compound 3 (2 µM) was concomitantly added to the cultures.

### Protection against the mitochondrial neurotoxin MPP⁺.

To determine whether compound 1 and compound 2 remained protective in a situation in which DA cell death was caused by mitochondrial poisoning with MPP⁺, the active metabolite of the DA neurotoxin MPTP was tested. For this purpose, spontaneously occurring DA cell death was prevented by a treatment combining depolarizing concentrations of K⁺ (30 mM) and MK801 (5 µM), a glutamate receptor antagonist used to prevent unwanted excitotoxic insult. The cultures were then exposed to 3 µM MPP⁺ between 5 and 7 DIV to achieve a loss of approximately 50% of DA neurons. When the cultures were exposed to compound 1 (30 µM) or compound 2 (1 µM) during the intoxication period, MPP⁺-induced DA cell loss was either partially or almost totally prevented by these two compounds, respectively **(****Figure 3****).** This effect was suppressed when the compound 3 (2 µM) was concomitantly added to the cultures.

### Protection from excitotoxicity.

Excitotoxicity occurring through an over activation of glutamate receptors is thought to play a key role in neurodegeneration (Lewerenz J, Maher P. Front Neurosci. 2015 Dec; 9:469). To determine whether compound 1 and compound 2 protected DA neurons from excitotoxic insults, we tested these compounds in a model in which neurodegeneration is induced by elevating extracellular K⁺ (30 mM), so as to produce a moderate and sustained increase in extracellular glutamate levels. We observed that cultures exposed to compound 1 (30 µM) or compound 2 (1 µM) were partially protected from excitotoxicity **(****Figure 4****)** and that this effect was suppressed when the compound 3 (2 µM) was concomitantly added to the cultures. Interestingly, compound 2 protected to a similar extent as the selective and uncompetitive NMDA glutamate receptor antagonist, memantine (8 µM).

### Rescue of neurons from oxidative stress.

Oxidative stress-mediated cell dysfunction has been shown to occur frequently in neurodegenerative pathologies (Chen X, et al. Neural Regen Res. 2012 Feb 15;7(5): 376-85.). Thus, a good neuroprotective agent should also possess anti-oxidant properties. To determine whether compound 1 and compound 2 presented this property, we applied chronically these compounds in a model in which degeneration results from a Fenton-type chemical reaction generating reactive oxygen species (Guerreiro S, et al. J Neurochem. 2009 May;109(4):1118-28). For this purpose, spontaneously occurring DA cell death was prevented by a treatment combining depolarizing concentrations of K⁺ (30 mM) and MK801 (5 µM), a glutamate receptor antagonist used to prevent unwanted excitotoxic insult. We observed that compound 1 (30 µM) or compound 2 (1 µM) protected DA neurons in this paradigm **(****Figure 5****).** Note that the protection conferred by compound 2 (1 µM) is similar in intensity to that conferred by the iron chelating and antioxidant agent desferrioxamine (DESF).

### Example 2: Anti-inflammatory effect of compound 1 and compound 2

### Limitation of the number of Microglial Cells.

Neuro-inflammation has been repeatedly implicated in neurodegeneration and microglial cells, the resident innate immune cells in the brain, may play a key role in this process. Since microglial cell activation generally starts with proliferation, we tested whether compound 1 or compound 2 were able to reduce the number of microglial cells *in vitro.* We observed that compound 1 (30 µM) or compound 2 (1 µM) partly reduced the number of microglial cells in 10 DIV midbrain cultures **(****Figure 6****)** and that this effect was suppressed when the compound 3 (2 µM) was concomitantly added to the cultures. Dexamethasone (DEX, 10 nM) was used as positive control, in this case.

### Example 3: Effect of compound 1 and compound 2 on degeneration of cortical neurons induced by excitotoxicity

As previously described compound 1 and compound 2 protected DA neurons of mesencephalic cultures from excitotoxic insults. To test whether this effect is limited to DA neurons, we reproduced this model using cortical neuron cultures that do not contain DA neurons. We observed that cultures exposed to compound 2 (1 µM) were partially protected from excitotoxicity **(****Figure 7****)** and that this effect was suppressed when compound 3 (2 µM) was concomitantly added to the cultures.

### Materials and methods

### Midbrain cell cultures

Animals were treated in accordance with the Guide for the Care and Use of Laboratory Animals (National Research Council, 1996), European Directive 86/609, and the guidelines of the local institutional animal care and use committee. Cultures were prepared from the ventral mesencephalon of gestational age 15.5 Wistar rat embryos (Janvier Breeding Center, Le Genest St Isle, France). Dissociated cells in suspension obtained by mechanical trituration of midbrain tissue pieces were seeded at a density of 1.2-1.5 10⁵ cells/ cm² onto tissue culture supports precoated with 1 mg/ml polyethylenimine diluted in borate buffer pH 8.3 as described (Michel PP, et al., J Neurochem. 1997 Oct;69(4):1499-507). The cultures were then maintained in N5 medium supplemented with 5 mM glucose, 5% horse serum, and 0.5% fetal calf serum, except for the first 3 days in vitro (DIV), when the concentration of fetal calf serum was 2.5% to favor initial maturation of the cultures (Guerreiro S, et al., Mol Pharmacol. 2008 Oct;74(4):980-9). They were fed daily by replacing 70% of the medium. Routinely, mesencephalic cultures were established on Nunc 24-well culture plates (Thermofischer Scientific, Rochester, NY). Note that these cultures contain tyrosine hydroxylase (TH)⁺ neurons that were exclusively dopaminergic (Traver S, et al., Mol Pharmacol. 2006 Jul;70(1):30-40). TH⁺ neurons represented approximately 1-2% of the total number of neuronal cells present in these cultures. The evaluation of the survival of DA neurons was performed by counting cells immunopositive for TH as described (Toulorge D, et al., Faseb J. 2011 Aug;25(8):2563-73).

### Culture systems used to model neuronal cell death

### Spontaneous dopaminergic (DA) cell death model and neuro-inflammation model.

We used a model system in which DA cell loss was spontaneous. The demise of DA neurons was progressive after plating to reach approximately 70-80% after 10 DIV (Guerreiro S, et al., Mol Pharmacol. 2008 Oct;74(4):980-9). For the spontaneous dopaminergic cell death model, TH⁺ neurons were counted while for the neuro-inflammation model, the number of microglial cells was evaluated.

### GDNF withdrawal model.

To evaluate the efficacy of our compounds in a more mature population of DA neurons, we also used a variation of the previous model system. More precisely, spontaneous DA cell death was prevented up to 10 DIV by a chronic treatment with GDNF (20 ng/mL) to favor the maturation of healthy DA neurons. Then, DA cell death was triggered by total withdrawal of GDNF for the next 5 days.

### MPP⁺ intoxication model.

Treatments with MPP⁺ were performed in cultures where the spontaneous death process was prevented by long-term exposure to depolarizing concentrations of K⁺ (30 mM), in the presence of the glutamate receptor antagonist MK801 (5 µM) to prevent unwanted excitotoxic insults as described previously (Douhou A, et al., J Neurochem. 2001 Jul;78(1):163-74). Treatments with MPP⁺ and potential neuroprotective molecules were carried out between 5 and 7 days in vitro (DIV), and the cultures were left to recover until 10 DIV in the presence of the control medium.

### Excitotoxicity model.

The treatment with elevated K⁺ (30 mM), which can trigger in itself excitotoxicity when applied without glutamate receptor antagonists (Douhou A, et al., J Neurochem. 2001 Jul;78(1):163-74), was added to midbrain or cortical cultures between 5 and 7 days in vitro (DIV) in the presence or the absence of potential neuroprotective molecules. The cultures were then left to recover until 10 DIV using control medium.

### Oxidative stress model.

Mesencephalic cells in suspension were plated onto polyethylenimine (1 mg/mL; Sigma-Aldrich) pre-coated culture plates (24 wells) and maintained in 500 µL of chemically defined serum-free medium consisting of equal volumes of Dulbecco's minimal essential medium and Ham's F12 nutrient mixture (Invitrogen). This medium was supplemented with 10 µg/mL insulin, 30 mM glucose and 100 U/mL penicillin and streptomycin. To favor cell attachment, fetal calf serum (10%) was also added to supplement the medium but only the first hour after plating. Cultures were fed every 2 days by replacing twice 350 µL of the culture medium.

### Quantification of neuronal survival and microglial cells number

The cultures were fixed for 12 min using 4% formaldehyde in Dulbecco's phosphate-buffered saline (PBS), then washed twice with PBS before an incubation step at 4 °C for 24 h with the following antibodies. A monoclonal anti-TH antibody diluted 1/5000 (ImmunoStar, Inc., Hudson, WI) or a polyclonal anti-TH antibody diluted 1/1000 (US Biologicals, Salem, MA) was used to assess the survival of DA neurons. A monoclonal anti-MAP2 antibody diluted at 1/200 (clone AP20, Sigma-Aldrich) was used to assess the survival of cortical neurons. Microglial cells were characterized using a mouse anti-CD11b antibody (1/50; clone MRC OX-42; Pharmingen, BD Biosciences, Le Pont-de-Claix, France). All antibodies were diluted in PBS containing 0.2% Triton X-100, except the mouse anti-CD11b which was diluted in PBS only. Detection of the primary antibodies was performed with an Alexa Fluor-488 conjugate of an anti-mouse IgG antibody or with an Alexa Fluor-555 conjugate of an anti-rabbit antibody (1:500).

### Cortical cell cultures

Animals were treated in accordance with the Guide for the Care and Use of Laboratory Animals (National Research Council, 1996), European Directive 86/609, and the guidelines of the local institutional animal care and use committee. Cultures were prepared from the cortex of gestational age 15.5 Wistar rat embryos (Janvier Breeding Center, Le Genest St Isle, France). Dissociated cells in suspension obtained by mechanical trituration of cortical tissue pieces were seeded onto tissue culture supports precoated with 1 mg/ml polyethylenimine diluted in borate buffer pH 8.3 according to the protocol previously described for mesencephalic cultures. The cultures were then maintained in N5 medium supplemented with 5 mM glucose, 5% horse serum, and 0.5% fetal calf serum, except for the first 3 days in vitro (DIV), when the concentration of fetal calf serum was 2.5% to favor initial maturation of the cultures (Guerreiro S, et al., Mol Pharmacol. 2008 Oct;74(4):980-9). They were fed daily by replacing 70% of the medium. Routinely, cortical cultures were established on Nunc 24-well culture plates (Thermofischer Scientific, Rochester, NY).

### Statistical Analysis

Simple comparisons between two groups were performed with Student's *t* test. Multiple comparisons against a single reference group were made by one-way analysis of variance followed by Dunnett's test. When all pairwise comparisons were required, the Student-Newman-Keuls test was used. S.E.M. values were derived from at least three independent experiments.

## Claims

1. A composition for use in treating a neurodegenerative disease comprising an agonist of the receptor of 1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid.

2. The composition for use according to claim **1,** wherein said agonist is selected from compounds of formula I, and triazine dyes; wherein formula I is: 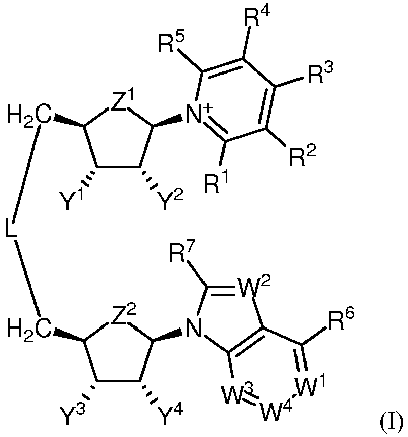 or a pharmaceutically acceptable salt, bioisostere or prodrug thereof, wherein
**Z¹** and **Z²** represent each independently O, S, CH₂ or a halo derivative thereof such as CF₂; preferably **Z¹** and **Z²** represent both O;
**Y¹**, **Y²** and **Y³** represent each independently a group selected from OH, H, NH₂, halo, SH, PO₄²⁻, O**R'** and -OC(O)**R'**, wherein **R'** represents hydrocarbyl; preferably **Y¹**, **Y²** and **Y³** all represent OH;
**Y⁴** represents a group selected from hydroxyl and -OP(O)(O**R"**)(O**R"'**) wherein **R"** and **R"'** represent each independently H, CH₂C(O)CH₃, CH₂OC(O)CH₃, a negative charge; or 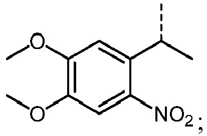 preferably **Y⁴** represents -OP(O)(OH)₂, -OP(O)(O⁻)₂ or -OP(O)(OCH₂OC(O)CH₃)₂;
or **Y³** and **Y⁴** are linked together and **-Y³-Y⁴-** represents -O-P(O)(OH)-O-;
**R¹** represents H;
**R²** represents a group selected from carboxylic acid or carboxylate, aminocarbonyl, hydroxyl, aldehyde, alkyl substituted by a carboxylic acid or a hydroxyl, alkyloxycarbonyl, alkylcarbonyloxyalkyloxycarbonyl; alkylcarbonylalkylcarbonyl, sulfate, phosphino, phosphono, boric acid and 1H-tetrazole; preferably **R²** represents carboxylic acid, carboxylate, or -CO-O-CH₂-O-CO-CH₃;
**R³** represents a group selected from H, alkyl, aryl and amino; preferably **R³** represents H.
**R⁴** represents a group selected from H, alkyl, hydroxyl, amino, carboxylic acid, alkylthio, azido and alkylcarbonylamido, wherein the alkyl group is optionally substituted by a group selected from hydroxyl, amino, azido, alkylcarbonylamido and alkyloxycarbonylamido; or **R⁴** is linked to **R⁵;** preferably **R⁴** represents H;
**R₅** represents H or is linked to **R⁵** so that **-R⁴-R⁵-** represents -CH=CH-CH=CH-;
**W¹**, **W²** and **W³** represent each independently CH or a heteroatom, such as N, P, S or O, preferably **W¹**, **W²** and **W³** all represent N;
**W⁴** represents CR⁸ or N; preferably **W⁴** represents CR⁸;
**R⁶** represents a group selected from amino, hydroxyl, deprotonated hydroxyl and thiol; or when **W¹** is N, **R⁶** is linked to **W¹** by an etheno link; preferably **R⁶** represents amino;
**R⁷** represents a group selected from H, amino, hydroxyl, thiol, halo (preferably Br) and azido; preferably **R⁷** represents H;
**R⁸** represents H; and
**L** represents a linker group of formula: 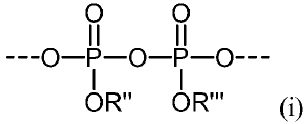 wherein **R"** and **R'"** represent each independently H, CH₂C(O)CH₃, CH₂OC(O)CH₃, a negative charge; or **L** may be selected from one or more of the group comprising: a phosphate, a polyphosphate, a phosphorothioate, a polyethylene glycol, an alkyl, an alkylaryl, a peptide and a polyamine; preferably **L** represents (i).

3. The composition for use according to claim **1** or claim **2,** wherein said agonist is selected from the group comprising:
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid;
1-((2R,3R,4S,5R)-5-((((((acetoxymethoxy)(((2R,3R,4R,5R)-5-(6-amino-9H-purin-9-yl)-4-((bis(acetoxymethoxy)phosphoryl)oxy)-3-hydroxytetrahydrofuran-2-yl)methoxy)phosphoryl)oxy)(hydroxy)phosphoryl)oxy)methyl)-3,4-dihydroxytetrahydrofuran-2-yl)-3-((acetoxymethoxy)carbonyl)pyridin-1-ium;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-sulfonic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryljoxymethyl] -3,4-dihydroxyoxolan-2-yl]pyridin-1-ium-3-acetic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]quinoline-1-yl-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-acetamido-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-hydroxy-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-(3-acetamidopropyl)-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-(3-t-Boc-aminopropyl)-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-(3-aminopropyl)-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-(3-hydroxypropyl)-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-(3-azidopropyl)-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-(thiomethyl)-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-4-methyl-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-methyl-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-amino-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-thiomethyl-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-4-amino-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-4-ethyl-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1 -ium-4-n-butyl-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-4-phenyl-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-carboxy-3-carboxylic acid;
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-azido-3-carboxylic acid;
1-[(2R,3R,4S,5R)-5-[[[[(2R,3R,4R,5R)-5-(6-amino-8-bromo-purin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid;
1-[(2R,3R,4S,5R)-5-[[[[(2R,3R,4R,5R)-5-(1,N⁶-etheno-adenine)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid;
1-[(2R,3R,4S,5R)-5-[[[[(2R,3R,4R,5R)-5-(1,N⁶-etheno-aza-adenine)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid;
1-[(2R,3R,4S,5R)-5-[[[[(2R,3R,4R,5R)-5-(6-amino-8-amino-purin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid;
1-[(2R,3R,4S,5R)-5-[[[[(2R,3R,4R,5R)-5-(6-amino-8-azido-purin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid;
1-[(2R,3R,4S,5R)-5-[[[[(2R,3R,4R,5R)-5-(purin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid;
1-[(2R,3R,4S,5R)-5-[[[[(2R,3R,4R,5R)-5-(6-amino-8-bromo-purin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-5-azido-3-carboxylic acid;
1-[(2R,3R,4S,5R)-5-[[[[(2R,3R,4R,5R)-5-(hypoxanthine)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxamide;
Reactive Red 120;
Reactive Green 19;
Reactive Green 5;
Cibacron Blue 3GA;
Reactive Yellow 86;
a bioisostere thereof; and/or a prodrug thereof.

4. The composition for use according to any one of claims **1** to **3,** wherein said agonist is selected from the group comprising:
1-[(2R,3R,4S,5R)-5-[[[[(2R,3R,4R,5R)-5-(6-aminopurin-9-yl)-3-hydroxy-4-phosphonooxyoxolan-2-yl]methoxy-hydroxyphosphoryl]oxy-hydroxyphosphoryl]oxymethyl]-3,4-dihydroxyoxolan-2-yl]pyridin-1-ium-3-carboxylic acid; and
1-((2R,3R,4S,5R)-5-((((((acetoxymethoxy)(((2R,3R,4R,5R)-5-(6-amino-9H-purin-9-yl)-4-((bis(acetoxymethoxy)phosphoryl)oxy)-3-hydroxytetrahydrofuran-2-yl)methoxy)phosphoryl)oxy)(hydroxy)phosphoryl)oxy)methyl)-3,4-dihydroxytetrahydrofuran-2-yl)-3-((acetoxymethoxy)carbonyl)pyridin-1-ium.

5. The composition for use according to any one of claims **1** to **4,** wherein said neurodegenerative disease is selected from the group comprising: Parkinson's disease and related disorders, motor neuron diseases, neuro-inflammatory diseases, Alzheimer's disease and related disorders, prion diseases, lysosomal storage diseases, leukodystrophies, Huntington's Disease, Down syndrome, spinal and bulbar muscular atrophy, HIV-Associated Neurocognitive Disorder, Tourette Syndrome, autosomal dominant spinocerebellar ataxia, Friedreich's Ataxia, Dentatorubral pallidoluysian atrophy, myotonic dystrophy, schizophrenia, age associated memory impairment, autism and autism spectrum disorders, attention-deficit hyperactivity disorder, chronic pain, alcohol-induced dementia, progressive non-fluent aphasia, semantic dementia, spastic paraplegia, fibromyalgia, post-Lyme disease, neuropathies, withdrawal symptoms, Alpers' disease, cerebro-oculo-facio-skeletal syndrome, Cockayne syndrome, Leigh's disease, neurodegeneration with brain iron accumulation, opsoclonus myoclonus syndrome, alpha-methylacyl-CoA racemase deficiency, Andermann syndrome, Arts syndrome, Marinesco-Sjögren syndrome, mitochondrial membrane protein-associated neurodegeneration, pantothenate kinase-associated neurodegeneration, polycystic lipomembranous osteodysplasia with sclerosing leukoencephalopathy, riboflavin transporter deficiency neuronopathy, and ataxia telangiectasia.

6. The composition for use according to claim **5,** wherein the Parkinson's disease and related disorders are selected from the group comprising Parkinson's disease, Parkinson-dementia, autosomal recessive PARK2 and PARK6-linked Parkinsonism, atypical parkinsonian syndromes, such as, for example, progressive supranuclear palsy, corticobasal degeneration syndrome, Lewy bodies dementia, multiple system atrophy, Guadeloupean Parkinsonism and Lytigo-bodig disease.

7. The composition for use according to claim **5,** wherein the motor neuron diseases are selected from the group comprising amyotrophic lateral sclerosis, frontotemporal dementia, progressive bulbar palsy, pseudobulbar palsy, primary lateral sclerosis, progressive muscular atrophy, spinal muscular atrophy and post-polio syndrome.

8. The composition for use according to claim **5,** wherein the Alzheimer's disease and related disorders are selected from the group comprising early stage of an Alzheimer's disorder, mild stage of an Alzheimer's disorder, moderate stage of an Alzheimer's disorder, mild to moderate stage of an Alzheimer's disorder, advanced stage of an Alzheimer's disorder, mild cognitive impairment, vascular dementia, mixed dementia, posterior cortical atrophy, Wernicke-Korsakoff Syndrome.

9. The composition for use according to claim **5,** wherein the neuro-inflammatory diseases are selected from the group comprising Alzheimer's disease, amyotrophic lateral sclerosis, multiple sclerosis, stroke, traumatic brain injury, HIV-associated neurocognitive disorder.

10. The composition for use according to claim **5,** wherein the prion diseases are selected from the group comprising Creutzfeldt-Jakob disease and its variants, variably protease- sensitive prionopathy, Gerstmann-Sträussler-Scheinker disease and fatal insomnia.

11. The composition for use according to claim **5,** wherein the lysosomal storage diseases are selected from the group comprising Sialidosis, Galactosialidosis, α-Mannosidosis, β-Mannosidosis, Aspartylglucosaminuria, Fucosidosis, Schindler disease, GM1 gangliosidosis, GM2-gangliosidosis, Tay-Sachs disease, Sandhoff disease, Gaucher disease, Metachromatic leukodystrophy, Multiple sulfatase deficiency, Globoid cell leukodystrophy, Fabry disease, Mucopolysaccharidoses, Pompe disease, Niemann-Pick, Farber lipogranulomatosis, Wolman and cholesteryl ester storage disease, Pycnodystostosis, Ceroide lipofuscinoses, Cystinosis, Salla disease, Danon disease, Chediak-Higashi disease, Griscelli diseases, and Hermansky Pudliak disease.

12. The composition for use according to claim **5,** wherein the neuropathies are selected from the group comprising peripheral neuropathy and diabetic neuropathy.

13. The composition for use according to claim **5,** wherein the leukodystrophies include without limitation, X-linked adrenoleukodystrophy, Adrenomyeloneuropathy, Alexander disease.
